# EUROPEAN PATENT APPLICATION

(11) **EP 3 928 726 A1**
(43) Date of publication of application: **29.12.2021**
(21) Application number: 21180595.7
(22) Date of filing: 21.06.2021
(51) Int. Cl.: A61B 17/74

(54) **SET SCREW FOR SETTING LAG SCREW STATE IN INTRAMEDULLARY NAIL SYSTEM AND INTRAMEDULLARY NAIL SYSTEM**

(30) Priority: 22.06.2020 CN 202010571724
(71) Applicant: Changzhou Kanghui Medical Innovation Co., Ltd., Changzhou, Jiangsu (CN)
(72) Inventor: GUAN, Jianjun, Changzhou, 213100 (CN); KE, Linhua, Changzhou, 213100 (CN); QIN, Zongjun, Changzhou, 213100 (CN)
(74) Representative: Müller-Boré & Partner Patentanwälte PartG mbB

(57) **Abstract**

The present disclosure relates to a set screw for setting a lag screw state in an intramedullary nail system, in particular for preventing the lag screw from protruding forwards out of the articular surface of the femur after surgery. The set screw is comprised of a driving member, a spring, and an engaging member; the driving member at a sidewall of an upper section is provided with external threads for cooperating with internal threads of an intramedullary main nail center hole; a sidewall at the lower end of the driving member is of an outer circular structure, where the outer circular structure is provided with a stepped outer circle, including: an engagement limiting segment at the lower part, an extended segment in the middle part, and a cooperation step at the upper part; the engaging member is of an outer circular structure provided with a guide boss at a sidewall, and the outer appearance of the guide boss is of a semi-circular shape; the engaging member at the upper part is provided with a slot, and the slot includes an upper slot which is a flange in clearance fit with the driving member extended segment, and a lower slot which is a chute in clearance fit with the driving member engagement limiting segment.

## Description

### FIELD

The present disclosure relates to a set screw, in particular, for setting a lag screw state in an intramedullary nail system, which can prevent damage to the articular surface caused by the lag screw protruding forwards from the femur after surgery. In addition, the present disclosure relates to an intramedullary nail system comprising the set screw.

### BACKGROUND

Intramedullary nailing is the main method for treatment of long bone fractures. In particular, more than 60% of the total amount of intramedullary nails are applied to treatment of intertrochanteric fractures of the thighbone. When choosing intramedullary nailing solutions for intertrochanteric fractures, surgeons typically take fracture types and bone conditions of patients into account as follows: a. for an unstable fracture, it is required to stably connect the main nail and the lag screw to form a stable internal fixing frame capable of providing a mechanical strength for rebuilding, thereby satisfying postoperative functional demands; b. for a stable fracture, it is required to form a sliding connection between the main nail and the lag screw, and the lag screw is allowed to move backwards in a limited distance along its axis, to compress the fracture ends to a limited extent to thus facilitate bone healing after surgery, and prevent the lag screw from protruding forwards out of the femur. In clinical practice, for a stable fracture, the existing products have the risk of causing the lag screw to protrude forwards although they can achieve limited withdrawal of the lag screw, when the main nail and the lag screw are in a locked state. Clinical failure of causing the lag screw to protrude forwards from the articular surface of the femur is a thorn in the surgeons' side.

### SUMMARY

In connection with the above defects, an objective of the present disclosure is to provide a set screw for setting a lag screw state in an intramedullary nail system, so as to prevent the lag screw from protruding forwards from the articular surface of the femur.

The set screw for setting a lag screw state in an intramedullary nail system comprises a driving member, an engaging member, and a spring. The driving member comprises a driving member proximal portion and a driving member distal portion. The driving member proximal portion has external threads for cooperating with internal threads of the main nail. The driving member distal portion is a cylinder having a diameter less than that of the driving member proximal portion. The engaging member comprises an engaging member proximal portion and an engaging member distal portion. The engaging member distal portion is provided with a protrusion for cooperating with a guide rail of the lag screw. After being installed in position, the spring engages the driving member at one end and engages the engaging member at the other end. By rotating the driving member, the lag screw can be switched between a locked state and an unlocked state. The external threads of the driving member proximal portion are configured to cooperate with internal threads within the guide pin through-hole at the proximal end of the main nail. The driving member distal portion is configured to receive the spring.

According to an aspect of the set screw of the present disclosure, the driving member distal portion comprises a spring limiting segment, an extended segment, and an engagement limiting segment, which are disposed coaxially and sequentially. The engaging member proximal portion comprises a slot having an axial opening and a radial opening. A radially inward flange is disposed in the slot and provided with a flange opening. An outer side surface of the flange is an abutment surface. The engagement limiting segment can enter the slot via the radial opening. When the extended segment moves after passing through the flange opening, an inner side surface of the flange limits movement of the engagement limiting segment within the engaging member proximal portion. The driving member distal portion is of a cylinder structure with a plurality of steps, which has an inverted "T" shape for matching an inverted "T" of the engaging member, thereby connecting the two members. The middle cylinder (i.e., the extended segment) in the middle of the driving member distal portion has a small outer diameter and thus is slidable within the slot of the engaging member proximal portion.

According to another aspect of the set screw of the present disclosure, the spring is a compression spring. The spring has a free length greater than a maximum installing distance of an inner end surface of the driving member proximal portion from the abutment surface of the engaging member proximal portion. The spring has a compression length less than a minimal installing distance of the inner end surface of the driving member proximal portion from the abutment surface of the engaging member proximal portion. The spring, which is a compression spring, is installed between the driving member and the engaging member, thereby enabling elastic sliding between the two members. A spring receiving space is provided between the inner end surface of the driving member proximal portion and the abutment surface of the engaging member proximal portion. The compression length of the spring is less than the minimal installing distance, the spring can thus be protected.

According to a further aspect of the set screw of the present disclosure, the protrusion of the engaging member distal portion and the guide rail of the lag screw are both toothed and configured to cooperate with each other. The protrusion may be a single tooth or have multiple teeth.

According to a still further aspect of the set screw of the present disclosure, a difference between the maximal installing distance and the minimal installing distance is greater than a height of the tooth of the guide rail.

According to a still further aspect of the set screw of the present disclosure, the slot has an axial length greater than a sum of the lengths of the extended segment and the engagement limiting segment.

According to a still further aspect of the set screw of the present disclosure, the driving member at an outer end is provided with a tool slot. The slot may be an inner hexagonal slot, torx slot, straight slot, or crossed slot, or may be a torx counterbore or other structure capable of providing rotational torque.

According to a still further aspect of the set screw of the present disclosure, the engaging member further comprises a guide boss disposed at one side of the engaging member proximal portion opposite to the slot. The guide boss is configured to match a guide slot within the center through-hole of the main nail, so as to orient the engaging member.

According to a still further aspect of the set screw of the present disclosure, the driving member is provided with a driving member pin guide through-hole. The engaging member is provided with an engaging member guide pin through-hole. After being installed in position, the driving member guide pin through-hole and the engaging member guide pin through-hole are coaxial. The driving member guide pin through-hole and the engaging member guide pin through hole allow a guide pin to pass through during surgery.

In connection with the above defects, a further objective of the present disclosure is to provide an intramedullary nail system. The intramedullary nail system comprises a main nail, a lag screw, and the set screw as described above. The main nail is provided with a guide pin through-hole. The set screw can be disposed within the guide pin through-hole, and the external threads of the driving member cooperate with internal threads of the guide pin through-hole. The protrusion of the engaging member is configured to cooperate with the guide rail of the lag screw.

According to an aspect of the intramedullary nail system of the present disclosure, the guide boss of the engaging member cooperates with a guide slot in the guide pin through-hole of the main nail.

According to another aspect of the intramedullary nail system, a tooth of the guide rail of the lag screw is a ratchet, which is configured to cooperate with the protrusion of the engaging member.

### Work principles:

According to the present disclosure, the set screw is installed within a main nail and configured to implement its function by setting an assembled state of a lag screw. During surgery, a driving member is rotated using a dedicated instrument to enable the set screw to move up and down within a center through-hole at the main nail proximal end. The positional relation between an engaging member and the lag screw is adjusted, and the lag screw is set in a locked or elastically locked state to implement an anticipated clinical function of stably locking the lag screw, or allowing advancement only.

The working state includes: for an unstable intertrochanteric fracture, the set screw is driven into a locked state, enabling formation of a stable structure from the lag screw and the intramedullary main nail, such that the set screw having an elastic structure can effectively prevent the lag screw from being loosened and guarantee a long-term locked state.

The working state includes: for a stable intertrochanteric fracture, the set screw is driven into a predetermined position, thereby elastically locking the lag screw with the intramedullary main nail. When a patient starts rehabilitation exercises, the head of the lag screw is subjected to longitudinal compressive stress as an effect of gravity and thus generate a stress stimulus to the fracture ends, which is beneficial to bone healing. Especially after two weeks since completion of the surgery, the fracture ends are at Bone Healing Stage II where absorption of the old bones occurs at the fracture ends, resulting in a gap between the two fracture ends, which may be even greater than 2 mm. In the circumstance, as an effect of gravity, the set screw according to the present disclosure reaches its elastic locking threshold. Then, the lag screw slides backwards along its axis to cause the fracture ends to be reclosed. Under the cyclical stress stimulus, the bones continue to grow until they are rejoined. In this process, the set screw according to the present disclosure satisfies the necessary clinical requirements by elastically locking the lag screw with the main nail. Moreover, the connection area between the head protrusion of the engaging member and the lag screw are configured with negative angles, to allow the lag screw to slide backwards only and thus effectively prevent the lag screw from protruding forwards from the articular surface of the femur. More specifically, in order to improve the anti-rotation performance of the lag screw, the lag screw is preferably fixed in a double-screw fashion. That is, a compression screw is installed in the lower hole of the overlapping hole at the proximal end of the main nail, to enable fixing in a double-screw fashion with the lag screw.

The set screw for setting a lag screw state in an intramedullary nail system according to the present disclosure has a simple structure and is convenient in use. The set screw is preferably pre-installed in an intramedullary main nail, which eliminates the assembling operation during surgery. What the surgeons need to do is only to choose the locking mode of the lag screw based on the patient's fracture type and bone condition, and then rotating the set screw according to the present disclosure to an anticipated position using a dedicated surgical instrument in light of the surgical requirements.

### BRIEF DESCRIPTION OF THE DRAWINGS

The present disclosure will be described below with reference to the drawings, where:
Fig. 1 is an exploded view of a structure of a set screw for setting a lag screw state in an intramedullary system;
Fig. 2 is a schematic diagram illustrating a structure of a driving member;
Fig. 3 is a schematic diagram illustrating a structure of an engaging member;
Fig. 4 is an assembling view of a set screw for setting a lag screw state in an intramedullary nail system;
Fig. 5 is a schematic diagram illustrating fixing, by a set screw for setting a lag screw state in an intramedullary nail system, a lag screw installed within the main nail;
Fig. 6 is an enlarged view of a match between an engaging member in the set screw and a toothed structure of the lag screw; and
Fig. 7 is an enlarged view of a match between the set screw and the main nail.

The drawings contain the following reference symbols for respective features, including:
- 1: driving member,
- 2: spring,
- 3: engaging member,
- 4: tail cap,
- 5: lag screw,
- 6: main nail,
- 7: locking screw,
- 8: compression screw;
- 1-1: external threads,
- 1-2: spring limiting segment,
- 1-3: extended segment,
- 1-4: engagement limiting segment,
- 1-5: driving member pin guiding through-hole,
- 1-6: tool slot,
- 1-7: inner end surface;
- 3-1: protrusion,
- 3-2: engaging member guide pin through-hole,
- 3-3: guide boss,
- 3-4: flange,
- 3-5: slot,
- 3-6: abutment surface;
- 5-1: guide rail;
- 6-1: center through-hole,
- 6-2: internal threads in center of main nail,
- 6-3: guide slot.

### DETAILED DESCRIPTION OF EMBODIMENTS

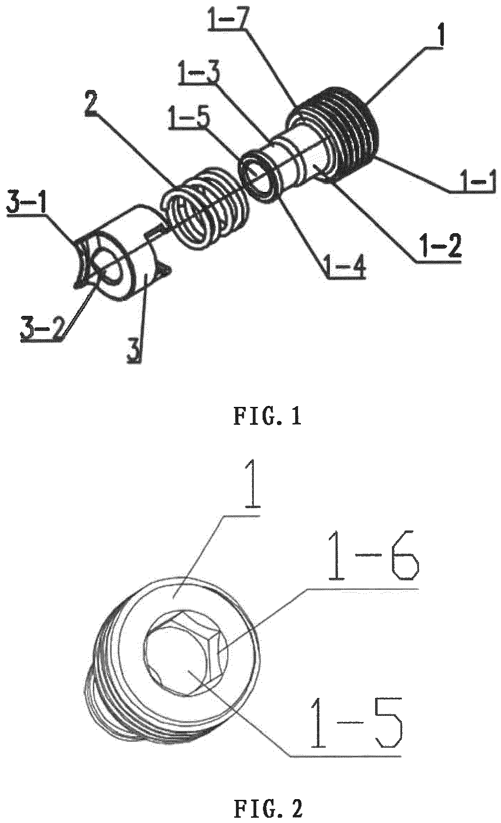

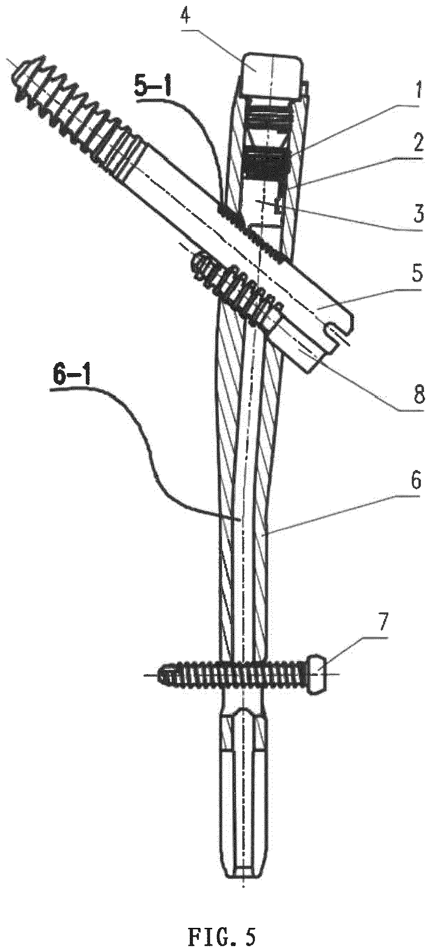

Through the following detailed description on example embodiments of the present disclosure with reference to Figs. 1-6, the above and other objectives, features, and advantages of the present disclosure will become more apparent, and wherein, the same reference symbols refer to the same elements.

For brevity, only parts related to the present disclosure are depicted in the respective drawings, which do not suggest the whole structure of the product. For further brevity of the drawings to facilitate understanding, only one is exemplarily depicted or marked therein for components having the same structure or function.

As described here, the term "one" not only represents "only one" but also represents "more than one."

Fig. 1 is an exploded view of a structure of a set screw for setting a lag screw state in an intramedullary system, wherein the set screw includes a driving member 1, a spring 2 and an engaging member 3.

In a further implementation, the driving member 1 is characterized in that the driving member at a proximal end surface is provided with a tool slot 1-6 for connection to a tool, which is preferably configured as an inner hexagonal counterbore, or may be configured as a torx counterbore or other structure capable of providing rotational torque, such as a straight or crossed slot. The driving member has external threads 101 at the proximal portion for matching internal threads 6-2 of a center through-hole of the main nail 6. The end surface of the proximal portion of the driving member facing the distal end is an inner end face 1-7 having a slot-like structure and configured to receive a spring. The driving member distal portion is of a cylinder structure having a plurality of steps, wherein an extended segment 1-3 and an engagement limiting segment 1-4 form an inverted "T" shape to match a slot 3-5 in an inverted "T" shape at the engaging member proximal portion, to thus connect the two members with each other. The extended segment 1-3 having a small outer diameter in the middle provides a space for receiving a spring 2, wherein a spring limiting segment 1-2 can reduce lateral displacement amplitude of the spring 2. The center of the driving member is a guide pin through-hole 1-5 which allows a guide pin to pass through during surgery.

In a further implementation, the engaging member 3 is characterized in that the main body of the engaging member 3 is cylindrical, and the engaging member 3 at the sidewall is provided with a semicircular guide boss 3-3 configured to match a guide slot 6-3 within the center through-hole 6-1 of the main nail 6, so as to orient the engaging member 3. The engaging member proximal portion is provided with an inverted "T" slot 3-5 at the sidewall for matching an engagement limiting segment 1-4 of the driving member in an inverted "T" shape. The engaging member is provided with an inner cavity at the middle portion to allow the engagement limiting segment 1-4 of the driving member in the inverted "T" shape to slide up and down. An axial opening of the engaging member proximal portion at the slot 3-5 is provided with a radially inward flange 3-4. The slot 3-5 has a length less than a compression length of the spring 2 to protect the length 2. The engaging member is provided with a protrusion 3-1 at a distal portion. The protrusion 3-1 may have a single-tooth or multi-teeth structure, preferably a single-tooth structure 3-1. The protrusion 3-1 cooperates with a guide rail 5-1 over a surface of a lag screw 5 of an intramedullary nail, which is preferably a ratchet. The center of the engaging member is a guide pin through-hole 3-2 which allows a guide pin to pass through during surgery.

In a further implementation, the spring 2 is preferably a compression spring. The spring 2 is installed between the driving member 1 and the engaging member 3 to achieve elastic sliding between the two members.

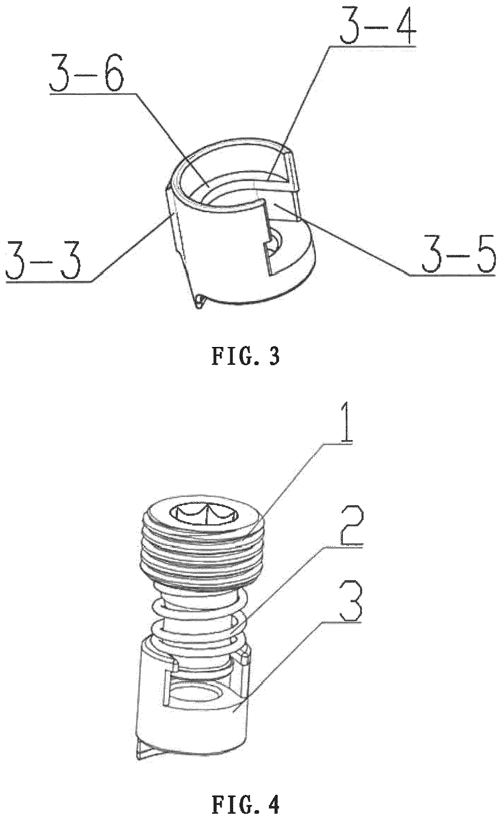

In a further implementation, the assembled state of the three components of the set screw according to the present disclosure is illustrated in Fig. 4, wherein the driving member 1, the spring 2 and the engaging member 3 are arranged sequentially from top to bottom. The three components are assembled as one piece having a compact structure and will not collapse. If the screw set is pressed from both the upper and lower end faces, the driving member 1 and the engagement 3 elastically slide relative to each other, and the elasticity involved therein is provided by the spring 2 disposed in the middle.

For better understanding on the function of the set screw according to the present disclosure, description will be provided hereinafter with reference to specific assembling examples of a femoral intramedullary nail.

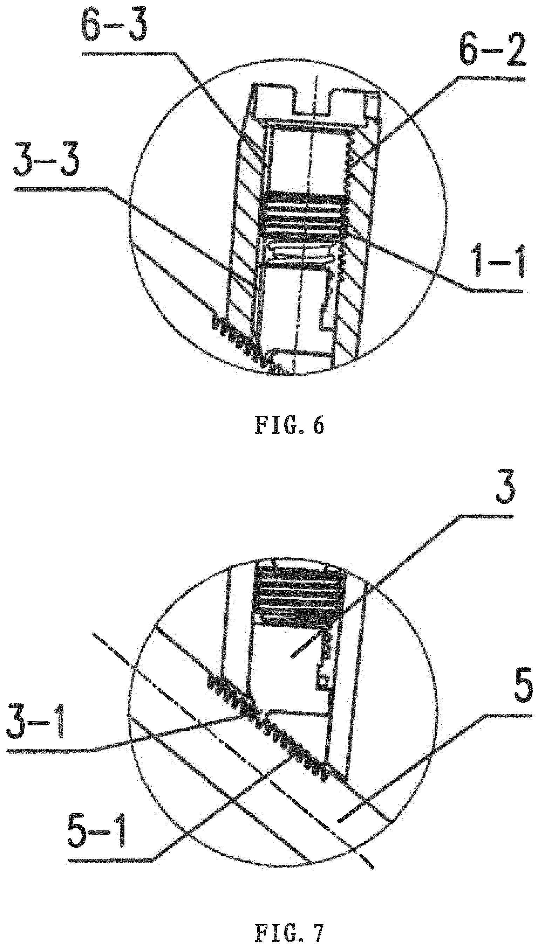

Embodiment 1: Fig. 6 is a schematic diagram illustrating a use state of a set screw for setting a lag screw state in an intramedullary nail system. The intramedullary nail system includes one driving member 1, one spring 2, one engaging member 3, one tail cap 4, one lag screw 5, one main nail 6, one locking screw 7, and one compression screw 8.

In a further implementation, the set screw according to the present disclosure is pre-installed in the center through-hole 6-1 at the proximal portion of the main nail 6. The semi-circular guide boss 3-3 on the sidewall of the engaging member 3 matches the guide slot 6-3 within the center through-hole 6-8 of the intramedullary main nail 6. The engaging member 3 may move up and down within the center through-hole 6-1 along the guide slot 6-3. The external threads 1-1 of the proximal portion of the driving member match the internal threads 6-2 within the center through-hole 6-1 of the main nail. After a tool is inserted into the tool slot 1-6 at the outer end surface of the driving member proximal portion during surgery, the driving member 1 is rotated to drive the whole set screw to move up and down in the center through-hole 6-1 along the guide slot 6-3.

In a further implementation, if the intertrochanteric fracture is an unstable type, the femoral intramedullary nail system needs to provide a strong internal fixing frame. At this time, the main nail 6 and the lag screw 5 need to form a stable locked state. In this circumstance, the driving member 1 is rotated by a tool to abut the engagement limiting segment 1-4 against the distal portion of the engaging member so as to set the set screw in a locked state, causing a stable connection formed between the lag screw 5 and the intramedullary main nail 6. In a stably locked state, the set screw of an elastic structure has an anti-loosening effect, thereby guaranteeing a long-term locked state.

Embodiment 2: Fig. 6 is a schematic diagram illustrating a use state of a set screw for setting a lag screw state in an intramedullary nail system. The intramedullary nail system includes one driving member 1, one spring 2, one engaging member 3, one tail cap 4, one lag screw 5, one main nail 6, one locking screw 7, and one compression screw 8.

In a further implementation, the set screw according to the present disclosure is pre-installed in the center through-hole 6-1 at the proximal portion of the main nail 6. The semi-circular guide boss 3-3 on the sidewall of the engaging member 3 matches the guide slot 6-3 within the center through-hole 6-8 of the intramedullary main nail 6. The engaging member 3 can move up and down within the center through-hole 6-1 along the guide slot 6-3. The external threads 1-1 of the proximal portion of the driving member match the internal threads 6-2 within the center through-hole 6-1 of the main nail. After a tool is inserted into the tool slot 1-6 at the outer end surface of the driving member proximal portion during surgery, the driving member 1 is rotated to drive the whole set screw to move up and down in the center through-hole 6-1 along the guide slot 6-3.

In a further implementation, if the intertrochanteric fracture is a stable type, elastic locking is preferably formed between the lag screw 5 and the intramedullary main nail 6, with the purpose of rejoining the fractured bones as an effect of limited withdrawal of the lag screw 5 within the overlapping hole of the intramedullary main nail 6 when a patient is at Bone Healing Stage II after surgery, to achieve benign healing under stress stimulation in the fracture area. The crucial point therein is that the screw set according to the present disclosure is rotated by a tool to a predetermined depth to form an elastic unlock between the lag screw 5 and the protrusion 3-1 of the engaging member distal portion.

In a further implementation, when the lag screw 5 is subjected to an axially downward thrust, the guide rail 5-1 at the rod portion of the lag screw 5 urges the upper toothed protrusion 3-1 of the engaging member 3 to rise in a certain height such that the engaged teeth temporarily disengage from each other. At this time, the lag screw 5 slides backwards by one pitch. The pattern is repeated until the fracture gap is closed.

In a further implementation, in the set screw, the protrusion 3-1 of the engaging member and the toothed rail 5-1 at the rod portion of the lag screw are configured with negative angles, i.e., ratchets, to allow the lag screw 5 to slide backwards only and thus effectively prevent the lag screw from protruding forwards from the articular surface of the femur.

In a further implementation, in order to improve the anti-rotation performance of the lag screw 5, the lag screw is preferably fixed in a double-screw fashion. That is, a compression screw 8 is installed in the lower hole of the overlapping hole at the proximal end of the intramedullary main nail 6, wherein the compression screw 8 and the lag screw 5 enable fixing in a double-screw fashion, thereby eliminating the degree of freedom of the lag screw 5 in terms of axial rotation.

## Claims

1. A set screw for setting a state of a lag screw in an intramedullary nail system, comprising:
a driving member (1) comprising a driving member proximal portion having external threads (1-1) for cooperating with internal threads of a main nail (6), and a driving member distal portion being a cylinder having a diameter less than that of the driving member proximal portion;
an engaging member (3) comprising an engaging member proximal portion and an engaging member distal portion having a protrusion (3-1) for cooperating with a guide rail of a lag screw (5); and
a spring (2);
wherein, after being installed in position, the spring (2) engages with the driving member (1) at one end and engages the engaging member (3) at the other end, and wherein, by rotating the driving member (1), the lag screw (5) can be switched between a locked state and an unlocked state.

2. The set screw for setting the lag screw state in the intramedullary nail system according to claim 1, wherein the driving member distal portion comprises a spring limiting segment (1-2), an extended segment (1-3), and an engagement limiting segment (1-4) disposed coaxially and sequentially; the engaging member proximal portion comprises a slot (3-5) having an axial opening and a radial opening; a radially inward flange (3-4) is disposed at the slot (3-5) and provided with a flange opening; an outer side surface of the flange (3-4) is an abutment surface (3-6), and the engagement limiting segment (1-4) can enter the slot (3-5) via the radial opening; when the extended segment (1-3) moves after passing through the flange opening, an inner side surface of the flange (3-4) limits movement of the engagement limiting segment (1-4) within the engaging member proximal portion.

3. The set screw for setting the lag screw state in the intramedullary nail system according to claim 2, wherein the spring (2) is a compression spring, a free length of the spring (2) being greater than a maximum installing distance of an inner end surface (1-7) of the driving member proximal portion from the abutment surface (3-6) of the engaging member proximal portion, and a compression length of the spring (2) being less than a minimal installing distance of the inner end surface (1-7) of the driving member proximal portion from the abutment surface (3-6) of the engaging member proximal portion.

4. The set screw for setting the lag screw state in the intramedullary nail system according to any of claims 1-3, wherein the protrusion (3-1) of the engaging member distal portion and the guide rail (5-1) of the lag screw are of a toothed shape and configured to cooperate with each other.

5. The set screw for setting the lag screw state in the intramedullary nail system according to claim 4, wherein the protrusion (3-1) is a single tooth or multiple teeth.

6. The set screw for setting the lag screw state in the intramedullary nail system according to claim 5, wherein a difference between the maximal installing distance and the minimal installing distance is greater than a height of tooth of the guide rail (5-1).

7. The set screw for setting the lag screw state in the intramedullary nail system according to claim 6, wherein the slot (3-5) has an axial length greater than a sum of the lengths of the extended segment (1-3) and the engagement limiting segment (1-4).

8. The set screw for setting the lag screw state in the intramedullary nail system according to claim 7, wherein the driving member is provided with a tool slot (1-6) at an outer end, the tool slot being an inner hexagonal slot, torx slot, straight slot, or crossed slot.

9. The set screw for setting the lag screw state in the intramedullary nail system according to claim 8, wherein the engaging member (3) further comprises a guide boss (3-3) disposed at one side of the engaging member proximal portion opposite to the slot (3-5).

10. The set screw for setting the lag screw state in the intramedullary nail system according to claim 9, wherein the driving member (1) is provided with a driving member pin guide through-hole (1-5), the engaging member (3) is provided with an engaging member guide pin through-hole (3-2), and after being installed in position, the driving member guide pin through-hole (1-5) and the engaging member guide pin through-hole (3-2) are coaxial.

11. An intramedullary nail system, comprising a main nail (6), a lag screw (5), and the set screw according to any of the preceding claims, wherein the main nail (6) is provided with a guide pin through-hole (6-1), the set screw can be disposed within the guide pin through-hole (6-1), the external threads (1-1) of the driving member (1) cooperate with internal threads of the guide pin through-hole (6-1), and the protrusion (3-1) of the engaging member (3) is adapted to cooperate with the guide rail (5-1) of the lag screw (5).

12. The intramedullary nail system according to claim 11, wherein the guide boss (3-3) of the engaging member (3) cooperates with a guide slot in the guide pin through-hole (6-1) of the main nail (6).

13. The intramedullary nail system according to claim 12, wherein a tooth of the guide rail (5-1) of the lag screw (5) is a ratchet and cooperates with the protrusion (3-1) of the engaging member (3).
